# EUROPEAN PATENT SPECIFICATION

(11) **EP 4 047 303 B1**
(45) Date of publication and mention of the grant of the patent: **07.08.2024**
(21) Application number: 22157847.9
(22) Date of filing: 21.02.2022
(51) Int. Cl.: G01B 9/02055, G01B 9/02091

(54) **OPTICAL COHERENCE TOMOGRAPHY-BASED SCANNER CALIBRATION DEVICE AND METHOD FOR OBTAINING CALIBRATION INFORMATION VIA A FULL AREA SCAN**
AUF OPTISCHER KOHÄRENZTOMOGRAPHIE BASIERENDE SCANNER-KALIBRIERUNGSVORRICHTUNG UND VERFAHREN ZUR GEWINNUNG VON KALIBRIERUNGSINFORMATIONEN ÜBER EINEN VOLLFLÄCHEN-SCAN
DISPOSITIF D'ÉTALONNAGE DE SCANNER BASÉ SUR LA TOMOGRAPHIE PAR COHÉRENCE OPTIQUE ET PROCÉDÉ D'OBTENTION D'INFORMATIONS D'ÉTALONNAGE VIA UN BALAYAGE DE ZONE COMPLÈTE

(30) Priority: 19.02.2021 KR 20210022386
(43) Date of publication of application: 24.08.2022
(73) Proprietor: Huvitz Co., Ltd., Anyang-si, Gyeonggi-do 14055 (KR); Ossvis Co., Ltd., Anyang-si, Gyeonggi-do 14055 (KR)
(72) Inventor: JEONG, Hyo Sang, 41430 Hwaseong Central Park 211-105, 100, Daecheon-ro, Buk-gu, Daegu (KR); CHO, Min Soo, 08087 (SM Elwy Apartment) 302, 25, Eunhaengjeong-ro 4-gil, Y-gu, Seoul (KR); LEE, Weon Joon, Dongan-gu, Anyang-si Gyeonggi-do, 14055 (KR)
(74) Representative: Wellburn, Daniel

(56) References cited:
- WO-A1-2020/208543
- US-A1- 2012 274 783
- US-B2- 9 593 933
- ZABIC MIROSLAV ET AL: "Wavefront sensorless adaptive optics for optical coherence tomography guided femtosecond laser surgery in the posterior eye", PROGRESS IN BIOMEDICAL OPTICS AND IMAGING, SPIE - INTERNATIONAL SOCIETY FOR OPTICAL ENGINEERING, BELLINGHAM, WA, US, vol. 10886, 20 February 2019 (2019-02-20), pages 1088603 - 1088603, XP060118734, ISSN: 1605-7422, ISBN: 978-1-5106-0027-0, DOI: 10.1117/12.2509363

## Description

### BACKGROUND

The present application claims priority to Korean Patent Application No. 10-2021-0022386 filed in the Korean Intellectual Property Office on 19 February 2021.

### FIELD

The present disclosure relates to an optical coherence tomography (OCT)-based scanner calibration device and a method for obtaining calibration information through full area scans, and more specifically, an optical coherence tomography-based scanner calibration device and a method for obtaining calibration information through full area scans that is capable of making use of an image sensor located on the front end of a calibration tip of the scanner to linearly scan the scan area covered by the scanner, based on the pixel information corresponding to the input voltages of the scanner.

### BACKGROUND

An OCT device is used to obtain images of an object in a contactless and noninvasive way. OCT is an imaging technology that is studied so as to solve problems of human (health) hazards, high cost, and measurement resolution associated with other types of measuring equipment such as an X-ray computed tomography (CT) machines, ultrasound imaging devices, and a magnetic resonance imaging (MRI) devices.

The OCT device obtains cross-section images in a micrometer unit through a Michelson interferometer and produces images with higher resolution than existing ultrasound images. Further, advantageously, the OCT device uses a near-infrared light source to measure the interior of an object in a non-invasive way. Furthermore, it is possible to obtain real-time OCT images, OCT devices be manufactured with a small-sized footprint and the cost of manufacture is relatively low.

The conventional OCT device does not typically obtain different OCT images of an object continuously. Therefore, if the OCT is to obtain the different OCT images, a plurality of optical couplers have to be used.

To solve such a problem, various calibration processes are needed. In conventional technologies, however, a target sample such as a lattice is used and an image analysis process is required for the calibration, which increases the complexity of the method. Furthermore, if the lattice is used, it is hard to distinguish an image of one area from another image of another area. As such, imaging errors may occur, and such errors cannot be detected easily.

US9593933B2 describes a method for calibrating and correcting the scanning distortion of any optical coherence tomography system by using reference patterns and obtaining mathematical relationships between the positions of the reference points in a reference pattern and the local coordinates of said reference points, said coordinates are obtained by means of said optical coherence tomography system.

### SUMMARY

Accordingly, the present disclosure has been made in view of the above-mentioned problems occurring in the related art, and it is an object of the present disclosure to provide an OCT-based scanner calibration device and method that is capable of linearly calibrating non-linear characteristics of an OCT-based scanner.

It is another object of the present disclosure to provide a method for obtaining calibration information for input voltages of an OCT-based scanner. The input voltages are those applied (to e.g. a scanner, such as a MEMS) for linearly scanning a scan area. The calibration is based on scan position information at measurement sample positions preferably across the full range of input voltages required to complete a scan over the full area which can be imaged by the scanner.

To accomplish the above-mentioned objects, according to one aspect of the present disclosure, there is provided an optical coherence tomography (OCT)-based scanner calibration device according to claim 1.

According to the present disclosure, desirably, the obtained pixel information may correspond to the position values of the measurement object.

To accomplish the above-mentioned objects, according to another aspect of the present disclosure, there is provided a method of obtaining calibration data for an optical coherence tomography (OCT)-based device according to claim 10.

### BRIEF DESCRIPTION OF THE DRAWINGS

The above and other objects, features and advantages will be apparent from the following detailed description of the preferred embodiments in conjunction with the accompanying drawings, in which:
FIG. 1 is a schematic of an OCT-based intraoral scanner on which a calibration tip is mounted, which is adopted in embodiments;
FIG. 2 is a graph showing the pixel information acquired through the calibration tip that corresponds to position information;
FIG. 3 is a flowchart showing a process of obtaining calibrated input voltages through full area scans according to embodiments;
FIG. 4 is a flowchart showing a method for capturing linearly calibrated scans with the use of calibrated input voltages as the input voltages of the OCT-based scanner; and
FIG. 5 is a flowchart showing another method for capturing linearly calibrated scans according to embodiments.

### DETAILED DESCRIPTION OF EMBODIMENTS

Objects, characteristics and advantages of the claimed invention will be more clearly understood from the detailed description as will be described below and the attached drawings. It is to be understood that the disclosed embodiments are merely exemplary, and the claimed invention can be embodied in various forms. Embodiments are described in detail so that the skilled person can more easily put the invention into practice. However, the embodiments do not limit the claims. The invention is defined by the scope of the appended claims.

Hereinafter, embodiments will be described and explained in detail with reference to the attached drawings.

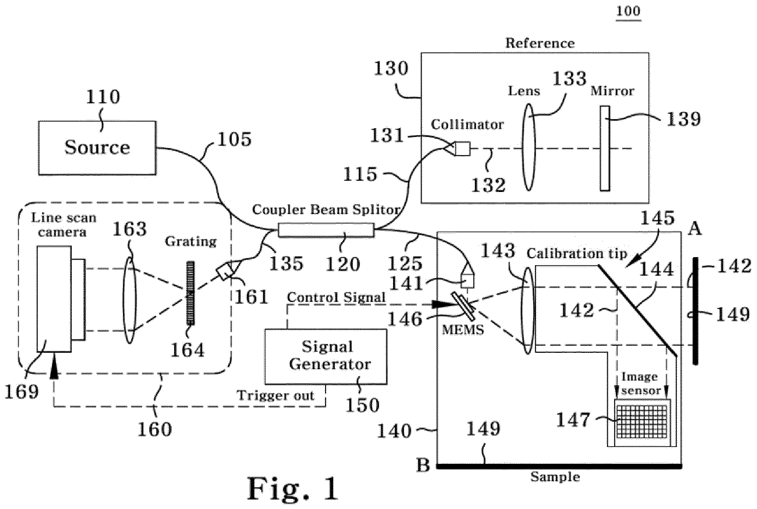

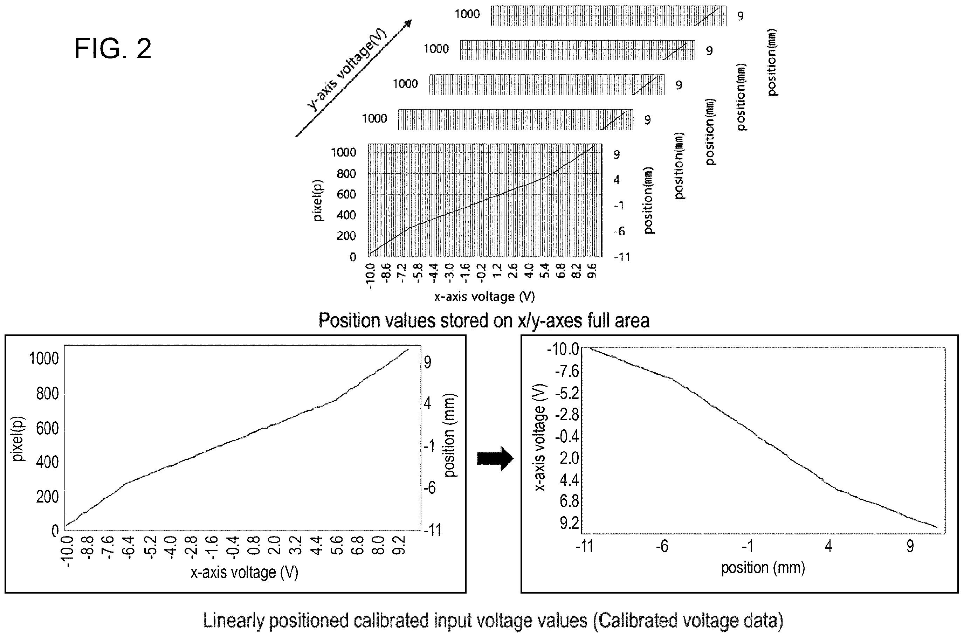

FIG. 1 is a schematic showing an OCT-based intraoral scanner on which a calibration tip is mounted, in accordance with embodiments, and FIG. 2 is a graph showing the pixel information acquired through the calibration tip that corresponds to position information.

Referring to FIGs. 1 and 2, an OCT-based intraoral scanner calibration device 100 according to an embodiment includes a light source 110, an optical coupler or beam splitter 120 arranged to split the path (or stream) of light irradiated from the light source into first light (or a first light stream) and second light (or a second light stream), a reference part 130 arranged to generate reference light from the first light split by the optical coupler or beam splitter, a sample part 140 for generating measurement light from the second light split by the optical coupler or beam splitter, a detector part 160 arranged to generate an OCT image from the reference light and the measurement light, and a signal generator 150 for generating a signal for controlling the sample part and synchronising the detector part 160.

It may be understood that in embodiments, the optical coupler or beam splitter 120 (hereinafter referred to as simply 'the optical coupler') is a 2x2 coupler/splitter having 2 first branches and 2 second branches. The optical coupler can operate in both coupling mode and splitting mode simultaneously depending on the connectivity of the 2 first and 2 second branches. However, the optical coupler is not limited thereto and can instead comprise a partially reflected mirror as is known in conventional OCT devices.

The light source 110 generates and emits light, and the light generated from the light source is transmitted to the optical coupler or beam splitter through at least one optical fiber 105. The light source 110 emits light having a wavelength in a near-infrared region, but it is not limited necessarily thereto.

In embodiments, the optical coupler 120 splits the path of the incident light through the at least one optical fiber 105 from the light source into the first light and the second light. The first light split through the optical coupler forms an optical input to the reference part 130, and the second light forms an optical input to the sample part 140. The first light split through the optical coupler or beam splitter is input to form a path 132 of the reference light, and the second light to form a path 141 of the measurement light.

According to embodiments, the first light emitted from the optical coupler 120 is input to the reference part 130 via at least one optical fiber 115 disposed between the optical coupler and the reference part, and the second light is input to the sample part 140 through at least one optical fiber 125 disposed between the optical coupler 120 and the sample part 140. The sample part 140 has a probe tip mounted thereon to capture (e.g. light reflected from) the sample. The sample can be, for example, teeth in an oral cavity.

The reference part 130 includes a collimator 131 arranged to receive the first light and output the reference light 132 from the at least one optical fibre 115, a lens 133 positioned to receive the reference light 132, and a mirror 139 arranged to receive light 132 from the lens 133 and reflect it back through the lens 133 and into the collimator 131.

The sample part 140 includes a collimator 141 arranged to receive the second light and output the measurement light 142 from the at least one optical fibre 115, a lens 143 positioned to receive the measurement light 132 from the collimator 141. The sample part includes a calibration tip including a prism 144. The prism can be arranged to reflect the measurement light 142 from the lens 143 and direct it towards a sample 149. The sample reflects the measurement light 142 back through the prism 144 and the lens 133 and into the collimator 131. The sample part 140 also includes a scanner 146 (e.g. a raster scanner such as a MEMS (microelectromechanical system)). The calibration tip includes an image sensor 147.

The detector part 160 includes a collimator 161 arranged to receive a combined (interference) signal of the reference light and the measurement light from the at least one optical fibre 135 connected between the collimator 161 and the optical coupler 120. The detector part 160 also includes a diffraction grating 164 arranged to receive the combined signal and separate the combined signal into a spectrum of wavelength separated signals. The wavelength separated signals are then received by a lens 163 which directs the signals onto a camera 169. The camera 169 may be a line scan camera arranged to build-up an image of the sample, one line at a time, or one pixel at a time, by capturing the combined signal when the MEMS scanner scans a line across the sample, or picks out a point on the sample.

The scanner 146 (e.g. scanning head) is configured to reflect the measurement light emitted from the collimator 141 onto the sample (e.g. via the lens 143 and prism 144 or via the lens 143 only). The scanner 146 receives scanning input voltages configured to control an angle of a reflective surface of the scanner relative to the incident measurement light, thereby controlling the angle of the measurement light output from the reflective surface of the scanner, thereby scanning or rastering the measurement light over the sample surface.

The scanning input voltages comprise a first scanning input voltage configured to control the position of the measurement light on the sample surface (or image sensor) in a first (e.g. y) direction and a second scanning input voltage configured to control the position of the measurement light on the sample surface (or image sensor) in a second (e.g. x) direction, wherein the first direction lies across (e.g. perpendicular to) the second direction.

The device 100 also includes a signal generator 150 arranged to send control signals to the scanner 146 and trigger signals to the camera 169 in the detector part 160 to synchronise movement of the MEMS with capturing of data (e.g. an image) from the sample using the camera 169. The signal generator 150 is communicatively coupled (e.g. via an electrical or wireless connection) to the MEMS 146 and the camera 169. A memory and processor may be connected to the signal generator.

In Fig 1. two alternative positions are shown for the sample 149. In a first position (A) the (surface of the) sample 149 faces the lens 143. In the first position, the prism 144 can include a partially reflective surface which reflects a portion of the measurement light toward the image sensor 147 and allows a portion of the measurement light 142 to be transmitted therethrough to the sample 149. In a second position (B), the sample 149 faces the output from the prism 144 after reflection from the reflective surface of the prism. In the second position, the image sensor 147 can be coupled to the prism 144 to detect the position of the measurement light 142 or can be removed from the prism so that the measurement light 142 irradiates the sample 149. In the first position, the image sensor can remain in place so as to capture or supply calibration information while the measurement light also irradiates the sample 149, because the image sensor 147 and sample 149 can be irradiated simultaneously. Alternatively, if the sample is in the first position and the prism does not allow light to pass through to the sample (by reflecting all light to the image sensor 147) the calibration tip (including the prism 144 and the image sensor 147) can be selectively removed or added to the sample part (i.e. the probe tip) so that the measurement light 142 can be scanned over the sample or image sensor, respectively, as desired.

In operation, the reference light 132 is generated from the first light input to the reference part 130, and the measurement light 142 from the second light input to the sample part 140. The reference light 132, which travels in a given (e.g. predetermined or known) path in the reference part 130 and is reflected from the mirror 139 and thereby output from the reference part. The reference light is thereby is input again to the optical coupler 120 via the at least one optical fiber 115 from which it originated. In normal use, the measurement light 142, which travels in a given path in the sample part 140, is reflected using the scanner 146 (and optionally the prism 144) onto a measurement object (e.g. the sample 149). The measurement light 142 is then reflected from the sample 149 back through the prism 144 and is output from the sample part 140. The measurement light 142 reflected from the sample is thereby input again to the optical coupler 120 via the at least one optical fiber 125 from which it originated. In the absence of the calibration tip 145, there is no prism and the lens 143 directs the measurement light 142 onto the sample directly.

The reference light 131 and the measurement light 142, which are input into the optical coupler 120 through the optical fibers 115, 125 from the reference part and the sample part, are input to the detector part 160 as a combined (or interference) signal through at least one optical fiber 135.

In normal operation of the OCT device, the detector part 160 generates the OCT images for the measurement object from the combined reference light and measurement light.

According to embodiments, the OCT device can operate in a calibration mode in which the sample part has a calibration tip mounted on the probe tip. The calibration tip 145 includes an image sensor 147. The calibration tip may also include the prism 144 as shown in Fig. 1, however, embodiments are not limited thereto. The prism 144 redirects (e.g. reflects) at least a portion of light from the lens 143 towards the image sensor 147. The image sensor 147 is mounted to the prism so that mounting of the calibration tip on the probe tip places the image sensor 147 reliably in the same position relative to the other components (e.g. the MEMS and/or lens 143) of the sample part 140. In some embodiments, the prism 144 may be replaced with a reflector (or partial reflector) and housing performing essentially the same function of allowing the image sensor 147 to be reliably mounted on the probe tip so that the scanner and the image sensor can be reliably positioned relative to each other in a predetermined relationship (i.e. so that their relative positions are fixed).

The image sensor 147 of the calibration tip 145 obtains pixel information corresponding to an area (e.g. full scan area) of the measurement object (e.g. sample 149) that can be scanned by the sample part 140. The pixel information and scanner input voltages are recorded as the scanner scans the image sensor pixels. The pixel information corresponds to the position information of the measurement object (e.g. sample) within the scan area.

FIG. 2 shows a series of plots of x-axis voltage (V) applied to the scanner vs pixel number in a first direction accross the image sensor. Within the series, the y-axis data voltage is gradually increased between plots so that a whole data set of x-y voltages vs pixel number are recorded.

As there is a given distance per one pixel (i.e. as the distance between pixels is known), the positional information of the measurement object (sample 149) can be acquired from the pixel information obtained from the image sensor 147. Therefore, the pixel information corresponding to the scanner input voltages is (or represents, or correlates with) the position information of the measurement object (See right hand ordinate axis of FIG. 2, which shows the position in mm relative to the centre of the image sensor).

Scanner input voltages for the MEMS can be obtained for an area (e.g. a full area) of the measurement object, wherein the scanner input voltages reliably correspond to known positions within the scan area. Therefore, the scanner input voltages required for scanning a particular point or line on the sample are calibrated based on the pixel information and scanner input voltages obtained during calibration using the calibration tip 145.

Further, the scanner input voltages corresponding to the position information obtained from the image sensor during calibration can be stored in a memory. The stored scanning input voltages can later be used to transform desired position information for a scan of a sample into to scanner input voltages. As shown in the plot in the lower left hand side of Figure 2, a the calibration voltage data is recorded as x-axis data voltage vs position. In the right hand side of Figure 2, this data can be transposed so that for a desired position of the scanned measurement light on the sample, an x-axis voltage can be obtained which will cause the scanner to direct the measurement light to that position. A reliably repeatable scan of the full area of a sample can be carried out using the stored scanner input voltages to direct the measurement light to calibrated positions throughout the full area on the sample (See FIG. 2).

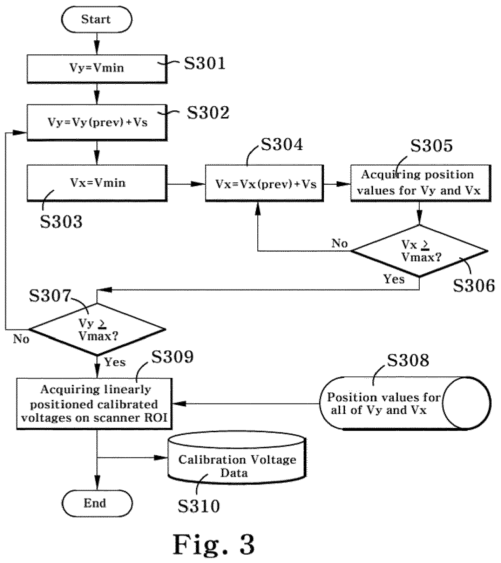

FIG. 3 is a flowchart showing a process of obtaining calibrated scanner input voltages through full area scans according to the present disclosure.

Referring to FIG. 3, in a first step S301, a first (y-axis) scanner input voltage Vy equals an initial value (Vy = Vmin). In a second step S302 the first (y-axis) scanner input voltage Vy is increased by one step voltage value Vs from its previous value Vy(prev) (in the first instance by one step voltage Vs from the initial value (Vy = Vmin)).

In a fourth step S304, a second (x-axis) scanner input voltage Vx equals an initial value (Vx = Vmin). In a fifth step S305, the second (x-axis) scanner input voltage Vx is increased by one step voltage value Vs from its previous value Vx(prev) (in the first instance by one step voltage Vs from the initial value (Vx = Vmin)).

In a fifth step S305, position values are acquired using pixel information obtained from the image sensor. This occurs when the measurement light is impingent upon one or more pixels in the image sensor, causing the pixel(s) to output a signal indicating that the measurement light irradiated the pixel. The location of the irradiated pixel(s) is also obtained. The first and second scanner input voltages Vx and Vy corresponding to the obtained location are stored in the memory along with the location of the irradiated pixel(s).

Whenever the first (y-axis) scanner input voltage is increased by one step voltage value (i.e. from the previous value or the initial value), the second (x-axis) scanner input voltage Vx is increased by one step voltage value from an initial value (Vx = Vmin). In a sixth step S306, there is a check as to whether the second (x-axis) scanner input voltage Vx has reached a maximum value Vmax. Vmin and Vmax may be the minimum and maximum voltages permitted by the extremes of the range of motion of the scanner 146. If the maximum voltage Vmax has not been reached, the process reverts to the fourth step S304 and the fourth and fifth steps are repeated until Vx reaches Vmax. Accordingly, the scanner input voltages corresponding to location values of pixels in the image sensor, which are generated until the second (x-axis) scanner input voltage is increased from a minimum input voltage to a maximum input voltage, are stored. The pixel location values measured by the image sensor correspond to the position values on the sample.

Once Vx reaches Vmax, a check is performed at a seventh step S307 as to whether the first (y-axis) scanner input voltage Vy has reached a maximum value Vmax. If Vy has not reached a maximum value, the first (y-axis) scanner input voltage Vy is increased again by one step voltage value, and in this case, the position values of the image sensor, which are generated until the second (x-axis) scanner input voltage is increased from a minimal input voltage to a maximum input voltage, are stored.

Until the first (y-axis) scanner input voltage Vy reaches the maximum voltage, the position values of the x-axis voltages according to the y-axis input voltages are repeatedly stored. Thus, once Vy has reached Vmax, the stepping up of the scanner input voltages ceases.

Through the repetition, in an eighth step S308, the position information for all of Vx and Vy can be obtained, and the position information is transformed into input voltages compared to the position information to thus, in a ninth step S309, linear position calibration voltages on a region of interest (ROI) is obtained. In a tenth step S310, the calibration voltage data is stored. That is, scanner input voltage information calibrated according to the positions detected by the pixels in the image sensor is stored.

As described above, the device further includes a memory arranged to store the pixel information from the image sensor. The memory is also arranged to store the first and second scanner input voltages.

The calibration voltage data provides the following benefits. First, the calibration voltages can be applied to the scanner to irradiate measurement light reliably and repeatedly at the same positions of a sample. Otherwise, the camera can be configured (e.g. triggered) so that it only samples the combined signal when the scanner input voltages match the calibration voltages. Thus, the same positions on the sample can be analysed each time. This allows multiple comparable images to be captured which OCT measurements are all provided according to the same positions on the sample. Alternatively, the input voltages applied to a scanner can be compared with the calibration voltages to detect whether the camera should sample the combined signal from the measurement object (See FIG. 2).

Further, it is possible to capture a scan of a sample (for example an oral scan) calibrated through the linear position calibration voltages obtained by the above-mentioned method.

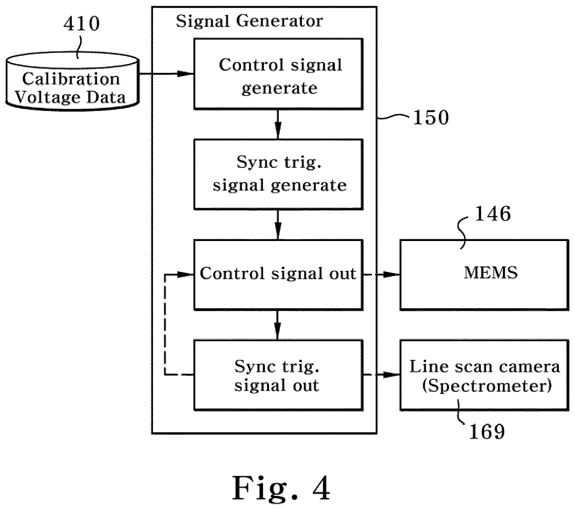

FIG. 4 is a flowchart showing a method for capturing a scan of a sample using the calibration voltage data (e.g. a calibrated oral scan) according to embodiments.

Referring to FIG. 4, voltage values (Vx and Vy values) to be input to the scanner 146 (in this case a micro-electromechanical system (MEMS)) are generated based on the calibrated voltage data 410 values.

A camera trigger signal is generated at a position corresponding to the generated voltage inputs to the MEMS.

Next, the voltage values are input to the MEMS, and the camera trigger signal is outputted to the camera 169 (e.g. a line scan camera) at the position corresponding to the voltage values inputted to the MEMS.

Through the above-mentioned process, the calibrated voltages are used as the input voltages of the OCT-based scanner, thereby making it possible to capture a linearly calibrated (oral) scan.

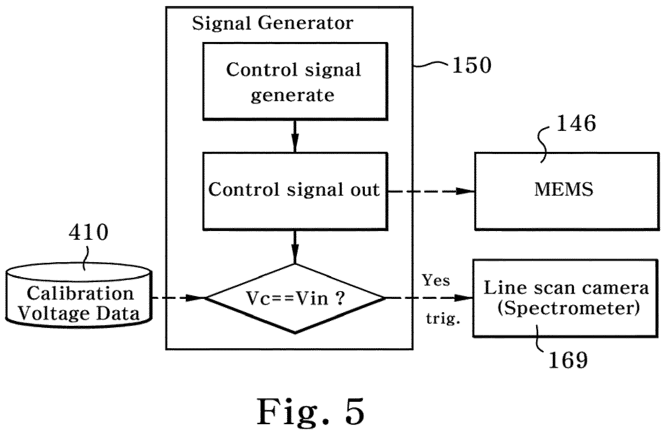

FIG. 5 is a flowchart showing another method for capturing linearly calibrated scans according to the present invention.

Referring to FIG. 5, first, a voltage waveform is generated by the signal generator 150 to operate a scanner 146 (e.g. a MEMS), and next, the generated voltage waveform is input to the MEMS. In this case, whenever the voltage value of the waveform inputted to the MEMS is equal to one of the calibrated voltage data values, the trigger signal is input to the camera 169 by the signal generator. In embodiments, the device further includes a processor arranged to compare the voltage value of the waveform with the calibration voltage data stored in the memory.

Accordingly, a voltage of a sine wave or sawtooth wave can be used as an input voltage for the OCT scanner, and if a current voltage corresponds to the calibrated input voltage, the trigger signal is generated to allow the camera to capture the linearly calibrated (oral) scan.

Although embodiments refer to an optical coherence tomography (OCT)-based scanner calibration device, it may be understood that the device may be applied as an intraoral scanner calibration device and as such all embodiments may be implemented as an optical coherence tomography (OCT)-based intraoral scanner calibration device. Although oral scans are described, the use of the devices and methods described herein are applicable to general OCT scanning applications.

As set forth in the foregoing, the present invention allows the pixel information of the image sensor to correspond to the position information, is intuitive so that calibration is easily performed, and replaces the input signal with the calibrated signal so that the scan result can be reliably reproduced each time and conveniently checked.

The foregoing description of the embodiments of the invention has been presented for the purpose of illustration; it is not intended to be exhaustive or to limit the invention to the precise forms disclosed. Persons skilled in the relevant art can appreciate that many modifications and variations are possible in light of the above teachings. It is therefore intended that the scope of the invention be limited not by this detailed description, but rather invention is defined by the scope of the claims appended hereto.

An alternative description of the Figures is also provided below
*FIG. 1* *is a concept view showing an OCT-based intraoral scanner on which a calibration tip is mounted, which is adopted in the present invention, and* *FIG.* 2 *is* a *graph showing the pixel information acquired through the calibration tip that corresponds to position information.*
*Referring to* *FIGs. 1* *and* *2**, an OCT-based intraoral scanner calibration device according to the present invention includes a light source, an optical coupler or beam splitter for splitting the path of light irradiated from the light source in first light and second light, a reference part for generating reference light from the first light split by the optical coupler or beam splitter, a sample part for generating measurement light from the second light split by the optical coupler or beam splitter, and* a *signal generator for generating an OCT image from the reference light and the measurement light.*
*The light source generates light, and the light generated from the light source is transmitted to the optical coupler or beam splitter through optical fibers. The light source emits light having a wavelength in a near-infrared region, but it is not limited necessarily thereto.*
*The optical coupler or beam splitter splits the path of the incident light through the optical fibers from the light source in the first light and the second light. The first light split through the optical coupler or beam splitter is inputted to the path of the reference light, and the second light to the path of the measurement light.*
*According to an embodiment of the present invention, the first light emitted from the optical coupler is inputted to the reference part through the optical fibers disposed between the optical coupler and the reference part, and the second light to the sample part through the optical fibers disposed between the optical coupler and the sample part. The sample part has* a *probe tip mounted thereon to capture the teeth scan in the oral cavity.*
*The reference light is generated from the first light inputted to the reference part, and the measurement light from the second light inputted to the sample part. The reference light, which moves by a given path in the reference part and is reflected and outputted therefrom, is inputted again to the optical coupler through the optical fibers. The measurement light, which moves by a given path in the sample part, is reflected onto a measurement object, and is outputted therefrom, is inputted again to the optical coupler through the optical fibers.*
*The reference light and the measurement light, which are incident onto the optical coupler through the optical fibers from the reference part and the sample part, are inputted to the signal generator through the optical fibers.*
*The signal generator generates the OCT images for the measurement object from the combined reference light and measurement light.*
*According to the present invention, in specific, the sample part has* a *calibration tip mounted on the probe tip and having an image sensor.*
*The image sensor of the calibration tip obtains pixel information corresponding to full area input voltages,* as *scanner input voltages. The pixel information corresponds to the position information of the measurement object.*
*As there is a given distance per one pixel, that is, the position information of the measurement object can be acquired from the pixel information obtained from the image sensor. According to the present invention, therefore, the pixel information corresponding to the input voltages is the position information of the measurement object (See* *FIG. 2**).*
*Further, the input voltages to the position information are transformed into the position information to input voltages to obtain the input voltages at the calibrated positions through full area scans (See* *FIG. 2**).*
*FIG. 3* *is a flowchart showing a process of obtaining calibrated input voltages through full area scans according to the present invention.*
*Referring to* *FIG. 3**, first, a y-axis input voltage is increased by one step voltage value from an initial value (Vy = Vmin).*
*In this case, whenever the y-axis input voltage is increased by one step voltage value from the initial value, an x-axis input voltage is increased by one step voltage value from an initial value (Vx = Vmin), and accordingly, the position values of the image sensor, which are generated until the x-axis input voltage is increased from a minimal input voltage to a maximum input voltage, are stored. This is because the pixel values measured by the image sensor correspond to the position values.*
*Next, the y-axis input voltage is increased again by one step voltage value, and in this case, the position values of the image sensor, which are generated until the x-axis input voltage is increased from a minimal input voltage to a maximum input voltage, are stored.*
*Until the y-axis input voltage reaches the maximum voltage, the position values of the x-axis full area voltages according to the y-axis input voltages are repeatedly stored.*
*Through the repetition, the position information for all of Vx and Vy can be obtained, and the position information is transformed into input voltages compared to the position information to thus obtain linear position calibration voltages on a region of interest (ROI), that is, voltage information calibrated according to the positions of the measurement object (See* *FIG. 2**).*
*Further, it is possible to capture the oral scan calibrated through the linear position calibration voltages obtained by the above-mentioned method.*
*FIG.* 4 *is* a *flowchart showing a method for capturing the calibrated oral scan according to the present invention.*
*Referring to* *FIG. 4**, voltage values (Vx and Vy values) to be inputted to micro-electromechanical systems (MEMS) are generated based on the calibrated voltage values.*
*A camera trigger signal is generated at a position corresponding to the generated voltage inputs to the MEMS.*
*Next, the voltage values are inputted to the MEMS, and the camera trigger signal is outputted at the position corresponding to the voltage values inputted to the MEMS. Through the above-mentioned process, the calibrated input voltages are used* as *the input voltages of the OCT-based intraoral scanner, thereby making it possible to capture the linearly calibrated oral scan.*
*FIG. 5* *is a flowchart showing another method for capturing linearly calibrated intraoral scans according to the present invention.*
*Referring to* *FIG. 5**, first, a voltage waveform is generated to operate the MEMS, and next, the generated voltage waveform is inputted to the MEMS. In this case, if the voltage value inputted to the MEMS is the calibrated voltage value, the trigger signal is inputted to a camera.*
*Accordingly, a voltage of a sine wave or sawtooth wave is used* as *an input voltage for the OCT scanner, and if a current voltage corresponds to the calibrated input voltage, the trigger signal is generated to allow the camera to capture the linearly calibrated oral scan.*
*As set forth in the foregoing, the present invention allows the pixel information of the image sensor to correspond to the position information, is intuitive so that calibration is easily performed, and replaces the input signal with the calibrated signal so that the calibration result can be conveniently checked.*

## Claims

1. An optical coherence tomography "OCT"-based scanner calibration device comprising:
a light source (110);
an optical coupler (120) arranged to split the light irradiated from the light source into first light and second light;
a reference part (130) arranged to generate reference light from the first light;
a sample part (140) arranged to generate measurement light from the second light; and
a detector part (160) arranged to generate an OCT image from the reference light and the measurement light,
**characterised in that**
the sample part comprises:
a calibration tip (145) having an image sensor (147), and
a scanner (146) arranged to scan the measurement light across the image sensor to generate pixel information; and
a memory arranged to store the pixel information and scanner input voltages corresponding to the positions of the measurement light on the image sensor,
wherein the pixel information and the scanner input voltages together define calibration voltage data for the scanner.

2. The device according to claim 2, wherein the calibration tip (145) is mounted on a probe tip of the device, wherein the probe tip is arranged to capture the measurement light after reflection from the sample.

3. The device according to claim 2, wherein the image sensor is arranged to obtain pixel information corresponding to an area of a sample (149) or measurement object, wherein the pixel information corresponds to input voltages of a scanner.

4. The device according to claim 2, wherein the scanner (146) is arranged to scan the measurement light over an area of a sample (149),
the image sensor includes an array of pixels and is positioned so as to:
match the position of the area of the sample relative to the scanned measurement light, and
receive the measurement light from the scanner.

5. The device according to any preceding claim, wherein the pixel information is based on:
at least one voltage generated as a result of measurement light irradiating at least one pixel of the image sensor,
the position of the at least one pixel.

6. The device according to any preceding claim, wherein the obtained pixel information corresponds to the positions in the area of the sample.

7. The device according to any preceding claim, further comprising a signal generator (150) arranged to supply scanner input voltages matching values in the calibration voltage data to the scanner.

8. The device according to claim 7, wherein the signal generator is arranged to supply a trigger signal to a camera (169) in the detector part so that the camera captures measurement data when the scanner input voltages match a value in the calibration voltage data.

9. The device according to any preceding claim, wherein the OCT-based scanner calibration device is an OCT-based *intraoral* scanner calibration device.

10. A method of obtaining calibration data for an optical coherence tomography "OCT"-based device, the method comprising:
scanning, using a scanner included in the device, measurement light across an image sensor included in a calibration tip,
acquiring pixel information from the image sensor during the scanning,
recording scanner input voltages for controlling the scanner, the scanner input voltages corresponding to the positions of the measurement light on the image sensor when the pixel information is acquired,
obtaining calibration voltage data using the scanner input voltages and the pixel information.

11. The method of claim 10, wherein the calibration voltage data is used to:
control scanner input voltages to match the calibration voltage data, and/or
trigger the operation of a camera when scanner input voltages matching the calibration voltage data are applied to the scanner.

12. The method of claim 10, further comprising the steps of:
increasing a first scanner input voltage by one step voltage value from an initial value Vy = Vmin;
whenever the first scanner input voltage is increased by one step voltage value from the initial value, increasing a second input voltage by one step voltage value from an initial value Vx = Vmin and storing the position values indicated by the image sensor generated until the second scanner input voltage is increased to a maximum input voltage;
until the first scanner input voltage reaches the maximum voltage, storing the position values across the whole area of the image sensor and the corresponding second scanner input voltages and first scanner input voltages to obtain the calibration voltage data.

13. The method according to any of claims 10 or 12, wherein, after the step of obtaining the calibrated voltage data, the method further comprises the steps of:
generating voltage values to be input to the scanner based on the calibrated voltage data;
generating a camera trigger signal at a position corresponding to the generated voltage inputs to the scanner;
inputting the voltage values to the scanner; and
outputting the camera trigger signal at the position corresponding to the voltage values inputted to the scanner.

14. The method according to claim 10 or 12, wherein after the step of obtaining the calibrated voltage data, the method further comprises the steps of:
generating a voltage waveform to operate the scanner;
inputting the generated voltage waveform to the scanner; and
if the voltage value input to the scanner matches the calibrated voltage data, inputting a trigger signal to a camera.

## Patentansprüche

1. Auf optischer Kohärenztomographie, "OCT", basierende Scanner-Kalibrierungsvorrichtung, umfassend:
eine Lichtquelle (110);
einen optischen Koppler (120), der zum Teilen des von der Lichtquelle ausgestrahlten Lichts in ein erstes Licht und ein zweites Licht angeordnet ist;
ein Referenzteil (130), das zum Erzeugen von Referenzlicht vom ersten Licht angeordnet ist;
ein Abtastteil (140), das zum Erzeugen von Messlicht vom zweiten Licht angeordnet ist; und
ein Detektorteil (160), das zum Erzeugen eines OCT-Bilds vom Referenzlicht und vom Messlicht angeordnet ist,
**dadurch gekennzeichnet, dass** der Abtastteil umfasst:
eine Kalibrierungsspitze (145), die einen Bildsensor (147) aufweist, und
einen Scanner (146), der zum Scannen des Messlichts über den Bildsensor zum Erzeugen von Pixelinformationen angeordnet ist; und
einen Speicher, der zum Speichern der Pixelinformationen und Scannereingangsspannungen entsprechend den Positionen des Messlichts am Bildsensor angeordnet ist,
wobei die Pixelinformationen und die Scannereingangsspannungen zusammen Kalibrierungsspannungsdaten für den Scanner definieren.

2. Vorrichtung nach Anspruch 2, wobei die Kalibrierungsspitze (145) an einer Sondenspitze der Vorrichtung befestigt ist, wobei die Sondenspitze zum Erfassen des Messlichts nach einer Reflexion von der Probe angeordnet ist.

3. Vorrichtung nach Anspruch 2, wobei der Bildsensor zum Gewinnen von Pixelinformationen entsprechend einer Fläche einer Probe (149) oder eines Messobjekts angeordnet ist, wobei die Pixelinformationen Eingangsspannungen eines Scanners entsprechen.

4. Vorrichtung nach Anspruch 2, wobei der Scanner (146) zum Scannen des Messlichts über eine Fläche einer Probe (149) angeordnet ist,
wobei der Bildsensor ein Array von Pixeln einschließt und angeordnet ist zum:
Abgleichen der Position der Fläche der Probe bezogen auf das gescannte Messlicht und
Empfangen des Messlichts vom Scanner.

5. Vorrichtung nach einem der vorstehenden Ansprüche, wobei die Pixelinformationen basieren auf:
mindestens einer Spannung, die dadurch, dass ein Messlicht mindestens ein Pixel des Bildsensors bestrahlt, erzeugt wird,
der Position des mindestens einen Pixels.

6. Vorrichtung nach einem der vorstehenden Ansprüche, wobei die gewonnenen Pixelinformationen den Positionen in der Fläche der Probe entsprechen.

7. Vorrichtung nach einem der vorstehenden Ansprüche, ferner umfassend einen Signalgenerator (150), der zum Bereitstellen von Scannereingangsspannungen, die mit Werten in den Kalibrierungsspannungsdaten übereinstimmen, an den Scanner angeordnet ist.

8. Vorrichtung nach Anspruch 7, wobei der Signalgenerator zum Bereitstellen eines Auslösesignals an eine Kamera (169) im Detektorteil angeordnet ist, sodass die Kamera Messdaten erfasst, wenn die Scannereingangsspannungen mit einem Wert in den Kalibrierungsspannungsdaten übereinstimmen.

9. Vorrichtung nach einem der vorstehenden Ansprüche, wobei die auf OCT basierende Scanner-Kalibrierungsvorrichtung eine auf OCT basierende *intraorale* Scanner-Kalibrierungsvorrichtung ist.

10. Verfahren zum Gewinnen von Kalibrierungsdaten für eine auf optischer Kohärenztomographie, "OCT", basierende Scanner-Kalibrierungsvorrichtung, das Verfahren umfassend:
Scannen, unter Verwendung eines Scanners, der in der Vorrichtung enthalten ist, von Messlicht über einen Bildsensor, der in einer Kalibrierungsspitze enthalten ist,
Erfassen von Pixelinformationen vom Bildsensor während des Scannens,
Erfassen von Scannereingangsspannungen zum Steuern des Scanners, wobei die Scannereingangsspannungen den Positionen des Messlichts auf dem Bildsensor, wenn die Pixelinformationen erfasst werden, entsprechen,
Gewinnen von Kalibrierungsspannungsdaten unter Verwendung der Scannereingangsspannungen und der Pixelinformationen.

11. Verfahren nach Anspruch 10, wobei die Kalibrierungsspannungsdaten verwendet werden zum:
Steuern von Scannereingangsspannungen, sodass sie mit den Kalibrierungsspannungsdaten übereinstimmen, und/oder
Auslösen des Betriebs einer Kamera, wenn Scannereingangsspannungen, die mit den Kalibrierungsspannungsdaten übereinstimmen, auf den Scanner angewendet werden.

12. Verfahren nach Anspruch 10, ferner umfassend die Schritte:
Erhöhen einer ersten Scannereingangsspannung um einen Schrittspannungswert von einem Anfangswert Vy = Vmin;
immer dann, wenn die erste Scannereingangsspannung um einen Schrittspannungswert vom Anfangswert erhöht wird, Erhöhen einer zweiten Eingangsspannung um einen Schrittspannungswert von einem Anfangswert Vx = Vmin und Speichern der Positionswerte, die durch den Bildsensor angezeigt und erzeugt werden, bis die zweite Scannereingangsspannung auf eine maximale Eingangsspannung erhöht wird;
bis die erste Scannereingangsspannung die maximale Spannung erreicht, Speichern der Positionswerte über die gesamte Fläche des Bildsensors und der entsprechenden zweiten Scannereingangsspannungen und ersten Scannereingangsspannungen zum Gewinnen der Kalibrierungsspannungsdaten.

13. Verfahren nach einem der Ansprüche 10 oder 12, wobei, nach dem Schritt des Gewinnens der kalibrierten Spannungsdaten, das Verfahren ferner die Schritte umfasst:
Erzeugen von Spannungswerten zur Eingabe in den Scanner basierend auf den kalibrierten Spannungsdaten;
Erzeugen eines Kameraauslösesignals an einer Position entsprechend den erzeugten Spannungseingaben in den Scanner;
Eingeben der Spannungswerte in den Scanner und
Ausgeben des Kameraauslösesignals an der Position entsprechend den Spannungswerten, die in den Scanner eingegeben werden.

14. Verfahren nach Anspruch 10 oder 12, wobei, nach dem Schritt des Gewinnens der kalibrierten Spannungsdaten, das Verfahren ferner die Schritte umfasst:
Erzeugen einer Spannungswellenform zum Betreiben des Scanners;
Eingeben der erzeugten Spannungswellenform in den Scanner und
wenn die Spannungswerteingabe in den Scanner mit den kalibrierten Spannungsdaten übereinstimmt, Eingeben eines Auslösesignals in eine Kamera.

## Revendications

1. Dispositif d'étalonnage de scanner à base de tomographie par cohérence optique « OCT », comprenant :
une source de lumière (110) ;
un coupleur optique (120) agencé pour diviser la lumière, irradiée depuis la source de lumière, en une première lumière et seconde lumière ;
une partie de référence (130) agencée pour générer une lumière de référence à partir de la première lumière ;
une partie d'échantillon (140) agencée pour générer une lumière de mesure à partir de la seconde lumière ; et
une partie de détection (160) agencée pour générer une image OCT à partir de la lumière de référence et de la lumière de mesure,
**caractérisé en ce que** la partie d'échantillon comprend :
une extrémité d'étalonnage (145) ayant un capteur d'image (147), et
un scanner (146) agencé pour scanner la lumière de mesure à travers le capteur d'image pour générer des informations de pixel ; et
une mémoire agencée pour stocker les informations de pixel et des tensions d'entrée de scanner correspondant aux positions de la lumière de mesure sur le capteur d'image,
dans lequel les informations de pixel et les tensions d'entrée de scanner définissent ensemble des données de tension d'étalonnage pour le scanner.

2. Dispositif selon la revendication 2, dans lequel l'extrémité d'étalonnage (145) est montée sur une extrémité de sondage du dispositif, dans lequel l'extrémité de sondage est agencée pour capturer la lumière de mesure après réflexion depuis l'échantillon.

3. Dispositif selon la revendication 2, dans lequel le capteur d'image est agencé pour obtenir des informations de pixel correspondant à une zone d'un échantillon (149) ou objet de mesure, dans lequel les informations de pixel correspondent à des tensions d'entrées d'un scanner.

4. Dispositif selon la revendication 2, dans lequel le scanner (146) est agencé pour scanner la lumière de mesure par-dessus une zone d'un échantillon (149),
le capteur d'image inclut un réseau de pixels et est positionné afin de :
assortir la position de la zone de l'échantillon relativement à la lumière de mesure scannée, et
recevoir la lumière de mesure provenant du scanner.

5. Dispositif selon une quelconque revendication précédente, dans lequel les informations de pixel sont sur la base de :
au moins une tension générée en conséquence de lumière de mesure irradiant au moins un pixel du capteur d'image, la position de l'au moins un pixel.

6. Dispositif selon une quelconque revendication précédente, dans lequel les informations de pixel obtenues correspondent aux positions dans la zone de l'échantillon.

7. Dispositif selon une quelconque revendication précédente, comprenant en outre un générateur de signal (150) agencé pour fournir des tensions d'entrée de scanner assorties à des valeurs dans les données de tension d'étalonnage au scanner.

8. Dispositif selon la revendication 7, dans lequel le générateur de signal est agencé pour fournir un signal de déclenchement à une caméra (169) dans la partie de détection pour que la caméra capture des données de mesure lorsque les tensions d'entrée de scanner sont assorties à une valeur dans les données de tension d'étalonnage.

9. Dispositif selon une quelconque revendication précédente, dans lequel le dispositif d'étalonnage de scanner à base d'OCT est un dispositif d'étalonnage de scanner intra-oral à base d'OCT.

10. Procédé d'obtention de données d'étalonnage pour un dispositif à base de tomographie par cohérence optique « OCT », le procédé comprenant :
le scannage, en utilisant un scanner inclus dans le dispositif, de lumière de mesure à travers un capteur d'image inclus dans une extrémité d'étalonnage,
l'acquisition d'informations de pixel à partir du capteur d'image durant le scannage,
l'enregistrement de tensions d'entrée de scanner pour commander le scanner, les tensions d'entrée de scanner correspondant aux positions de la lumière de mesure sur le capteur d'image lorsque les informations de pixel sont acquises,
l'obtention de données de tension d'étalonnage en utilisant les tensions d'entrée de scanner et les informations de pixel.

11. Procédé de la revendication 10, dans lequel les données de tension d'étalonnage sont utilisées pour :
commander des tensions d'entrée de scanner pour qu'elles soient assorties aux données de tension d'étalonnage, et/ou
déclencher le fonctionnement d'un caméra lorsque des tensions d'entrée de scanner sont assorties aux données de tension d'étalonnage sont appliquées au scanner.

12. Procédé de la revendication 10, comprenant en outre les étapes de :
l'augmentation d'une première tension d'entrée de scanner selon une valeur de tension en échelon à partir d'une valeur initiale Vy = Vmin ;
à chaque fois que la première tension d'entrée de scanner est augmentée selon une valeur de tension en échelon à partir de la valeur initiale, l'augmentation d'une seconde tension d'entrée selon une valeur de tension en échelon à partir d'une valeur initiale Vx = Vmin et le stockage des valeurs de position indiquées par le capteur d'image générées jusqu'à ce que la seconde tension d'entrée de scanner soit augmentée jusqu'à une tension d'entrée maximum ;
jusqu'à ce que la première tension d'entrée de scanner atteigne la tension maximum, le stockage des valeurs de position à travers la zone entière du capteur d'image et des secondes tensions d'entrée de scanner et premières tensions d'entrée de scanner correspondantes pour obtenir les données de tension d'étalonnage.

13. Procédé selon l'une quelconque des revendications 10 ou 12, dans lequel, après l'étape de l'obtention des données de tension étalonnées, le procédé comprend en outre les étapes de :
la génération de valeurs de tension destinées à être entrées dans le scanner sur la base des données de tension étalonnées ;
la génération d'un signal de déclenchement de caméra à une position correspondant aux entrées de tension générées pour le scanner ;
l'entrée des valeurs de tension dans le scanner ; et
la sortie du signal de caméra de déclenchement à la position correspondant aux valeurs de tension entrées dans le scanner.

14. Procédé selon la revendication 10 ou 12, dans lequel, après l'étape de l'obtention des données de tension étalonnées, le procédé comprend en outre les étapes de :
la génération d'une forme d'onde de tension pour faire fonctionner le scanner ;
l'entrée de la forme d'onde de tension générée, dans le scanner ; et
si la valeur de tension entrée dans le scanner est assortie aux données de tension étalonnées, l'entrée d'un signal de déclenchement dans une caméra.
